Europäisches Patentamt
European Patent Office
Office européen des brevets

(19)

(11) Publication number: **0 336 705 A2**

(12) **EUROPEAN PATENT APPLICATION**

(21) Application number: 89303306.8

(22) Date of filing: 04.04.89

(51) Int. Cl.[4]: **C 07 D 501/26**
C 07 D 501/36, A 61 K 31/545

(30) Priority: 05.04.88 JP 83580/88

(43) Date of publication of application: 11.10.89 Bulletin 89/41

(84) Designated Contracting States: AT BE CH DE ES FR GB GR IT LI LU NL SE

(71) Applicant: AJINOMOTO CO., INC.
5-8, Kyobashi 1-chome, Chuo-ku
Tokyo 104 (JP)

(72) Inventor: Hayashi, Sadao
No 6-21 Shimizu-cho
Ashiya-shi Hyogo-ken (JP)

Mizutani, Akihito
B42-101 No 1 Ishishiro Otokoyama,
Yawata-shi Kyoto-hu (JP)

(74) Representative: Harrison, David Christopher et al
MEWBURN ELLIS & CO 2/3 Cursitor Street
London EC4A 1BQ (GB)

# (54) Cephem compounds and a production method therefor.

(57) The invention relates to cephem compounds or salts thereof of the formula:

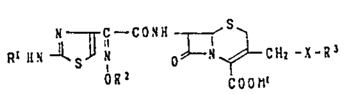

(I)

[in the formula, $R^1$ denotes hydrogen or an amino protective group, $R^2$ denotes an unsubstituted or substituted alkyl group, X denotes an O-bonded group or S-bonded group, and $M^1$ denotes hydrogen, carboxyl protective group or a cation and, as $COOM^1$, a carboxylate ion, and $R^3$ denotes a group represented by the formula:

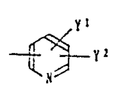

(IIa)

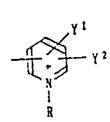

(IIb)

or

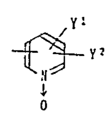

(IIc)

(in the formulae, $Y^1$ and $Y^2$ are identical or different and denote hydrogen, alkyl groups, halogen groups, hydroxyl groups or oxo groups, R denotes an alkyl group, and the bonding partner of the -X-group is positioned in the 1-position, 3-position or 4-position of the pyridine ring (but excluding the 1-position in the case of the pyridinium ring and the pyridine N-oxide ring)) (but excluding groups represented by the formula (II b) when X is an S-bonded group,], methods of making such compounds and antimicrobial agents incorporating them.

EP 0 336 705 A2

## Description

# Cephem compounds and a production method therefor.

Detailed description of the invention

Field of industrial use

This invention relates to novel cephem compounds and to a production method therefor. More specifically, it relates to cephem compounds which have an antimicrobial activity and which are useful as antimicrobial agents, and to a production method therefor.

Prior art, problems to be overcome by the invention

By way of cephem compounds having an antimicrobial activity, there has been, for example, a report in the laid-open Japanese patent application (Kokai) No. 61-134390 of compounds having a pyridylthiomethyl group in the 3-position of the cephem ring. However, these are compounds having 1-alkylpyridylthiomethyl groups, and compounds having 1-oxypyridylthiomethyl groups have not been synthesized. Furthermore, compounds having 1-alkylpyridyloxymethyl groups have not been synthesized either.

Furthermore, compounds having 1-oxy-2-pyridylthiomethyl groups in the 3-position of the cephem ring have been reported in the laid-open West German Patent (OLS) No. 2,716,707. However, there is no report of the antimicrobial activity of the said compounds in this.

An object of this invention is to provide cephem compounds having a high antimicrobial activity with respect to many pathogenic microorganisms and a production method therefor.

Another object of this invention is to provide cephem compounds which are useful as intermediates for the aforementioned cephem compounds and a production method therefor.

A further object of this invention is to provide antimicrobial agents containing, as an active principle, cephem compounds or salts thereof with a high antimicrobial activity.

Means of overcoming the problems

This invention relates to cephem compounds or salts thereof represented by the formula:

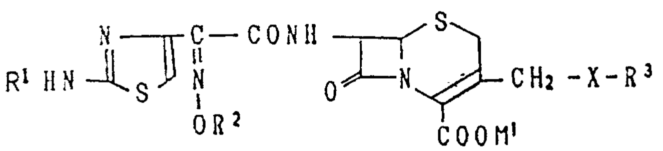

(I)

[in the formula, $R^1$ denotes hydrogen or an amino protective group, $R^2$ denotes an unsubstituted or substituted alkyl group, X denotes an O-bonded group or S-bonded group, and $M^1$ denotes hydrogen, a carboxyl group protective group or a cation and, as $COOM^1$, a carboxylate ion, and $R^3$ denotes a group represented by the formula:

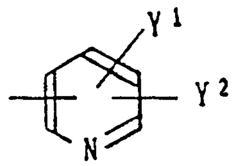

(IIa)

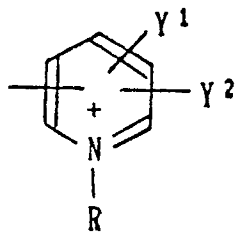

(IIb)

or

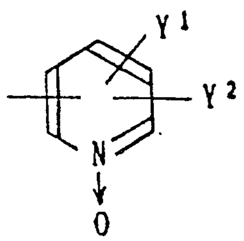

(IIc)

(in the formulae, $Y^1$ and $Y^2$ are identical or different and denote hydrogen, alkyl groups, halogen groups, hydroxyl groups or oxo groups, R denotes an alkyl group, and the bonding partner of the -X- group is positioned in the 1-position, 3-position or 4-position of the pyridine ring (but excluding the 1-position in the case of the pyridinium ring and the pyridine N-oxide ring)) (but excluding groups represented by the formula (II b) when X is an S-bonded group,].

Furthermore, this invention relates to a production method for cephem compounds represented by formula (I) or salts thereof, characterized in that a cephem compound represented by the formula:

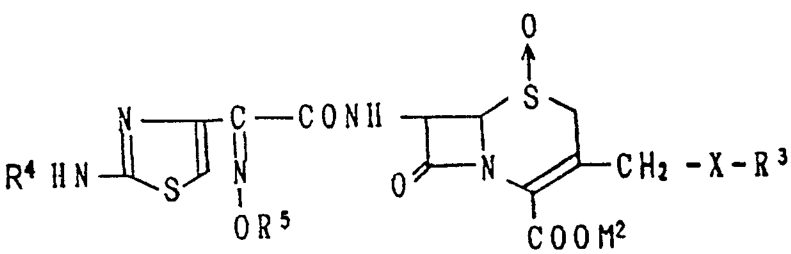

(III)

[in the formula, $R^4$ denotes an amino protective group, $R^5$ denotes an unsubstituted or substituted alkyl group (but protected by a protective group when the substituent group of the substituted alkyl group is a reactive group), $M^2$ denotes a carboxyl protective group, and X and $R^3$ respectively denote the same groups as mentioned previously] is subjected to a deoxidation reaction to obtain a cephem compound represented by the formula:

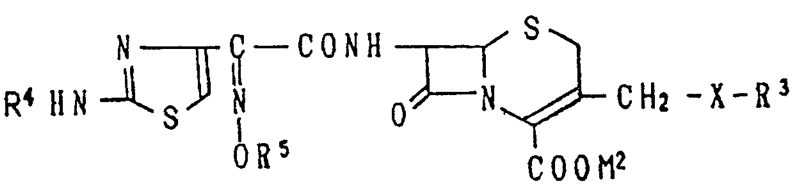

(IV)

(in the formula, $R^4$, $R^5$, $M^2$, X and $R^3$ respectively denote the same groups as mentioned previously), in other words the protective groups are eliminated.

Furthermore, this invention relates to a production method for cephem compounds represented by formula (I) and salts thereof, characterized in that 3-halogenomethylcephem compounds represented by the formula:

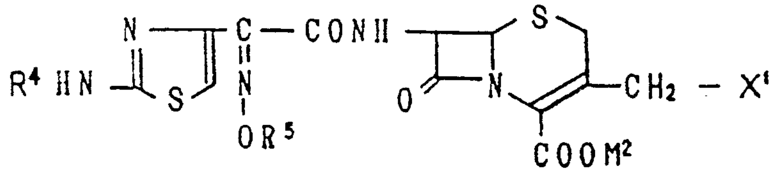

(V)

(in the formula, $X^1$ denotes a halogen group, and $R^4$, $R^5$ and $M^2$ respectively denote the same groups as mentioned previously) are reacted with hydroxy- or mercapto-pyridine derivatives represented by the formula:
H-X-$R^3$ (VI)
(in the formula, X and $R^3$ respectively denote the same groups as mentioned previously), to obtain a cephem compound represented by the formula:

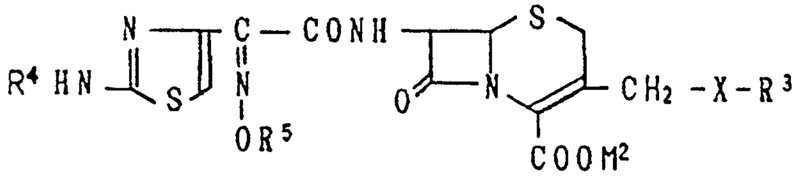

(IV)

(in the formula, $R^4$, $R^5$, $M^2$, X and $R^3$ respectively denote the same groups as mentioned previously), in other words the protective groups are eliminated.

Moreover, this invention relates to cephem compounds of salts thereof represented by the formula:

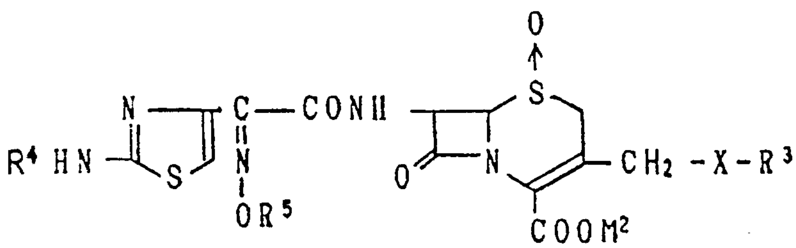

(III)

(in the formula, $R^4$, $R^5$, $M^2$, X and $R^3$ respectively denote the same groups as mentioned previously).

Moreover, this invention relates to a production method for cephem compounds represented by formula (III) and salts thereof, characterized in that a 3-halogenomethylcephem-1-oxide compound represented by the formula:

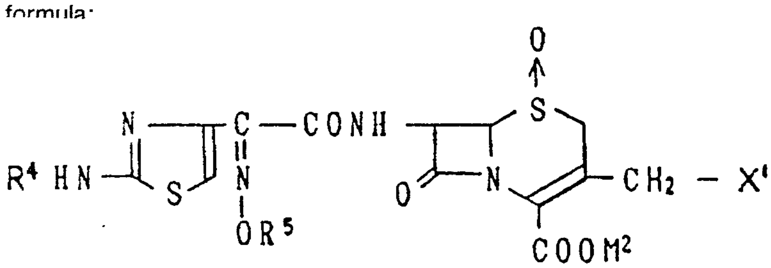

(VII)

(in the formula, $R^4$, $R^5$, $M^2$ and $X^1$ respectively denote the same groups as mentioned previously) is reacted with a hydroxy- or mercapto-pyridine derivative represented by the formula:
H-X-$R^3$ (VI)
(in the formula, X and $R^3$ respectively denote the same groups as mentioned previously).

Moreover, this invention relates to antimicrobial agents containing a cephem compound represented by the aforementioned formula (I) or salt thereof as the active principle.

Examples

The cephem compounds of this invention are substances represented by the formula:

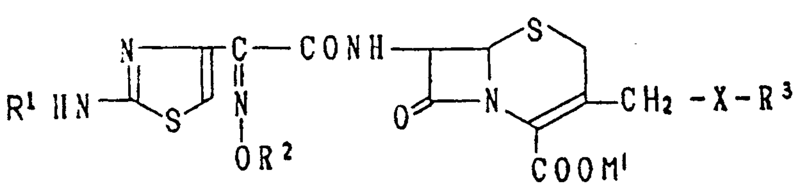

(I)

[in the formula, $R^1$ denotes hydrogen or an amino protective group, $R^2$ denotes an unsubstituted or substituted alkyl group, X denotes an O-bonded group or S-bonded group, and $M^1$ denotes hydrogen, a

carboxyl group protective group or cation and, as $COOM^1$, a carboxylate ion, and $R^3$ denotes a group represented by the formula:

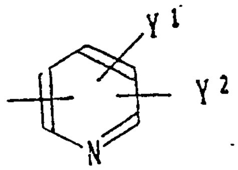

(IIa)

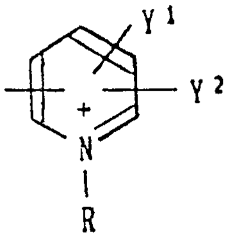

(IIb)

or

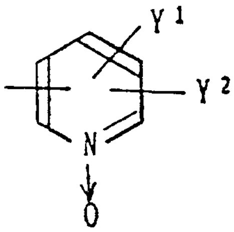

(IIc)

(in the formulae, $Y^1$ and $Y^2$ are identical or different and denote hydrogen, alkyl groups, halogen groups, hydroxyl groups or oxo groups, R denotes an alkyl group, and the bonding partner of the -X- group is positioned in the 1-position, 3-position or 4-position of the pyridine ring (but excluding the 1-position in the case of the pyridinium ring and the pyridine N-oxide ring)) (but excluding groups represented by the formula (II b) when X is an S-bonded group,].

Alkyl groups with 1 to 5 carbon atoms are, for example, preferred as the alkyl groups shown by $R^2$ in the aforementioned formula (I), and it is specifically possible to mention methyl, ethyl, n-propyl, isopropyl, cyclopropyl, n-butyl, i-butyl, t-butyl, n-pentyl and the like.

Alkyl groups with 1 to 5 carbon atoms which have been substituted with 1 or 2 or more substituent groups such as, for example, the hydroxyl group, carboxyl group, halogen group, carbamoyl group, carboxyamido group, amino group and N-oxide group are preferred as the substituted alkyl groups represented by $R^2$, and it is specifically possible to mention 2-hydroxypropyl, carboxymethyl, carbamoylmethyl,1-methyl-1-carboxyethyl, difluoromethyl, 2-aminoethyl, N-oxydimethylaminoethyl and the like.

$R^3$ in formula (I) is a group represented by

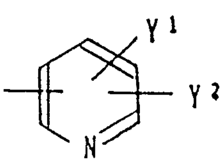

,

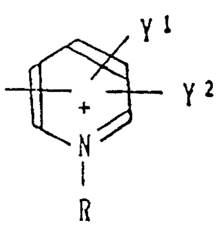

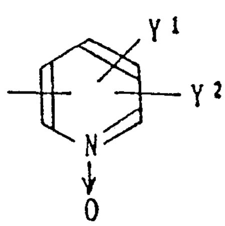

(II a) (II b) (II c)

Alkyl groups with 1 to 5 carbon atoms are preferred as the alkyl groups shown by $Y^1$ and $Y^2$ in $R^3$, and a chloro group or fluoro group is preferred as the halogen group. Alkyl groups with 1 to 5 carbon atoms are preferred as R in formula (II b).

Specific examples of the group $-X-R^3$ include, for example,

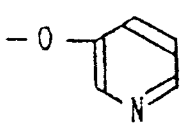 、 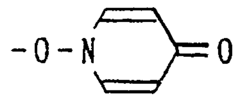 、 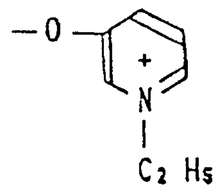 、

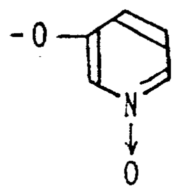 、 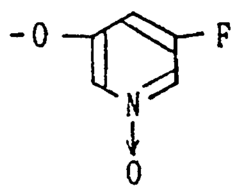 、 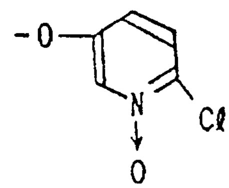 、

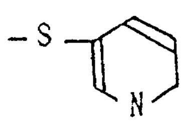 、 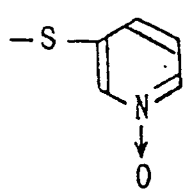 、 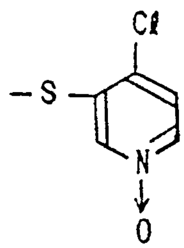 、

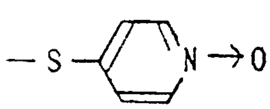 、 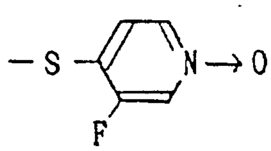 、

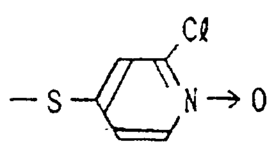 、 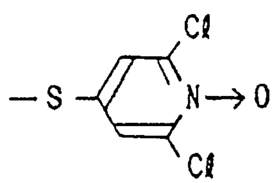

and the like.

Any of the normally used amino protective groups can be used as the amino protective group shown by $R^1$ in formula (I); for example, butoxycarbonyl, benzyloxycarbonyl, formyl, chloroacetyl and other such groups with carboxyl radicals, and benzyl, methoxybenzyl, ethoxy benzyl, trityl and other such aralkyl groups.

Furthermore, by way of the carboxyl protective group shown by $M^1$, it is possible to use any of the carboxyl protective groups which are known in the field of penicillin and cephalosporin chemistry as detachable groups which do not cause detrimental changes in other moieties. By way of example, it is possible to mention groups which represent aralkyl groups (for example, benzyl, methylbenzyl, dimethylbenzyl, methoxybenzyl, ethoxybenzyl, nitrobenzyl, aminobenzyl, diphenylmethyl, phthalidyl and phenacyl), unsubstituted or substituted alkyl groups (for example, trichloroethyl, t-butyl and allyl), unsubstituted or substituted aryl groups (for example, pentachlorophenyl and indanyl), groups derived from compounds having a N-hydroxyimino group (for example, groups derived from acetoneoxime, acetophenoneoxime, acetaldoxime, N-hydroxysuccinimide, N-hydroxyphthalimide and the like), trialkylsilyl groups (for example, trimethylsilyl), and acid anhydrides which react with carboxyl groups and are derived from carbonic acid or a carboxylic acid [for example,

$-COO\underset{\underset{O}{\|}}{C}-ORa$, $-COO\underset{\underset{O}{\|}}{C}-Ra$

(here, Ra denotes a lower alkyl group)]. Substances which are highly reactive towards substituted amido groups, substituted hydrazino groups and the like are also included amongst the carboxyl protective groups as referred to in this invention.

In particular, useful carboxyl protective groups are the protective groups which provide pharmacologically active esters exhibiting a strong antimicrobial activity upon oral or non-oral administration and which are well known as substances appropriate to medical use. By way of such protective groups, it is possible to use alkanoyloxyalkyl groups (for example, pivaloyloxymethyl, acetoxymethyl, 1-(acetoxy)ethyl), alkoxyformyloxyalkyl groups (for example, 1-(1-propyloxycarbonyloxy)ethyl, 1-(cyclohexyloxycarbonyloxy)ethyl) and other such substituted alkyl groups; phenacyl, phthalidyl and other such substituted aralkyl groups; phenyl, xylyl, indanyl and other such unsubstituted or substituted aryl groups and the like. By way of the cation shown by $M^1$, it is possible to mention the ions of sodium, potassium and other such alkali metals, the ions of calcium, magnesium and other such alkaline earth metals, and the ammonium ions of trimethylamine, triethylamine, pyridine, picoline, dicyclohexylamine, N,N-dibenzylethylenediamine and the like.

By way of salts of the compound (I) of this invention (meaning the salts of moieties apart from $COOM^1$) it is possible to mention the salts of acetic acid, trifluoroacetic acid, maleic acid, tartaric acid, methanesulfonic acid, benzenesulfonic acid, formic acid, toluenesulfonic acid and other such organic acids, the salts of hydrochloric acid, hydrobenzoic acid, hydriodic acid, sulphuric acid, phosphoric acid and other such inorganic acids, and the salts of arginine aspartic acid, glutamic acid and other such amino acids.

Compounds in which $R^2$ in formula (I) is a methyl group or 2-hydroxy-1-propyl group are preferred as the compound (I) of this invention.

Moreover, compounds in which X is an O-bonded group and $R^3$ is a group in which $Y^1$ and $Y^2$ are both hydrogen or a group in which $Y^1$ is hydrogen and $Y^2$ is a 4-oxo group in formula (II a), a group in which R is an ethyl group and $Y^1$ and $Y^2$ are both hydrogen in formula (II b), or a group in which $Y^1$ and $Y^2$ are both hydrogen in formula (II c) or compounds in which X is a S-bonded group and $R^3$ is a group in which $Y^1$ and $Y^2$ are both hydrogen, or $Y^1$ is hydrogen and $Y^2$ is a fluoro group or chloro group in formula (II c) are preferred.

In the compound (I) of this invention, the structure of the 2-aminothiazole group moiety is in an equilibrium state of a tautomer shown by the equilibrium equation below.

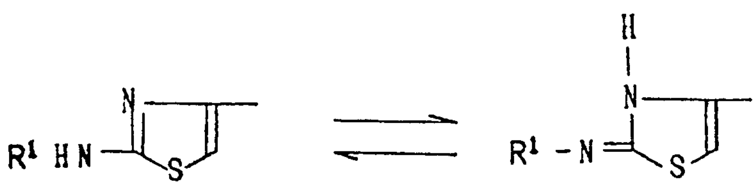

(A) (A′)

It is well known that both the tautomers are in an equilibrium relationship and in a mutually changeable state, and such tautomers can be regarded as essentially the same compound. In this invention as well, the above-mentioned tautomers (A) and (A′) are both contained within the scope of compound (I). Moreover, for convenience, the source compounds and the target compounds containing the abovementioned tautomer groups in this invention are shown using the method of expressing one of these tautomers, in other words the 2-aminothiazole group formulation and formula (A).

Furthermore, in the structure of the moiety shown by the formula: $=N\text{-}OR^2$ in the compound (I) of this invention, there are geometric isomers based on the presence of the double bond, in other words the syn-isomer and the anti-isomer shown below.

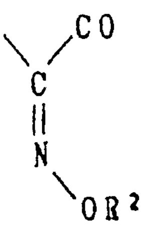

Syn

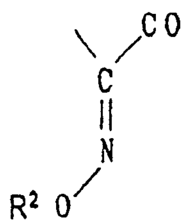

Anti

The compound (I) of this invention includes the respective syn-isomers and anti-isomers given above and mixtures thereof.

The compound (I) of this invention can be produced by the following methods.

Production method 1

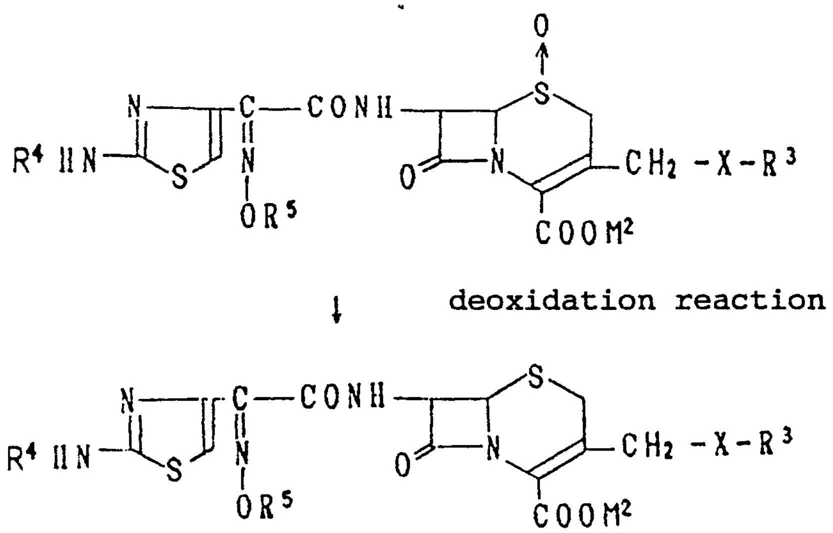


(III)

(IV)

protective group eliminating reaction as required

Compound (I)

In formulae (III) and (IV), $R^4$ denotes an amino protective group, $R^5$ denotes an unsubstituted or substituted aralkyl group, and $M^2$ denotes a carboxyl protective group, and X and $R^3$ respectively denote the same groups as mentioned previously.

The same groups as the amino protective groups shown by $R^1$ in the abovementioned formula (I) are used as the amino protective groups shown by $R^4$.

The same groups as the unsubstituted or substituted alkyl groups shown by $R^2$ in the abovementioned formula (I) are used as the unsubstituted or substituted alkyl groups shown by $R^5$. When a substituent group of the substituted alkyl group is a reactive group, it is protected by a protective group. $R^5$ is a substance containing a substituted alkyl group protected by a protective group in this way. A protective group such as a trialkylsilyl group (for example, trimethylsilyl) is used when the substituent group of the substituted alkyl group is a hydroxyl group; a trityl group, chloroacetyl group, t-butyoxycarbonyl group when it is an amino group and, for example; a biphenylmethyl group, p-methoxybenzyl group or p-nitrobenzyl group when it is a carboxyl group.

The same groups as the carboxyl protective group shown by $M^1$ in formula (I) mentioned previously are used as the carboxyl protective group shown by $M^2$.

The reaction in which compound (IV) is obtained by reducing the -SO- group in compound (III) to a -S-group can be carried out in the presence of a common reducing agent, for example phosphorus trichloride or other such phosphorus halide. Furthermore, it can be carried out using the action of acetyl chloride in the presence of potassium iodide.

The compound (IV) so obtained can be turned into a cephem compound with a high antimicrobial activity by means of an elimination reaction for the amino protective group, carboxyl protective group or other such protective group as required.

All the customary methods can be appropriately used in the elimination of the carboxyl protective group; for example, methods such as elimination reactions using hydrolysis, reduction ($H_2$-Pd/C etc.), or a Lewis acid (for example, trifluoroacetic acid or aluminium chloride). It is preferable that this elimination reaction is carried out in the presence of a cation-trapping agent such as anisole, phenol or the like. Usually, it is carried out in a solvent such as nitromethane, methylene chloride, diethyl ether or carbon disulphide, but it is possible to carry it out in the presence of any other solvent provided the solvent does not exert any adverse influence on the reaction. Furthermore, it is possible to carry it out in a mixture of these solvents.

It is possible to appropriately use all of the customary methods in the amino protective group elimination reaction. For example, the reaction can be carried out using 80% by weight of formic acid or trifluoroacetic acid or the like with respect to the trityl group or butoxycarbonyl group, using thiourea with respect to the chloroacetyl group, using a reducing agent (for example, hydrogen reduction using Zn-acetic acid or Pd/C catalysts) or the like with respect to the benzyloxycarbonyl group, or using methanol-hydrochloric acid or the like with respect to the formyl group.

Production method 2

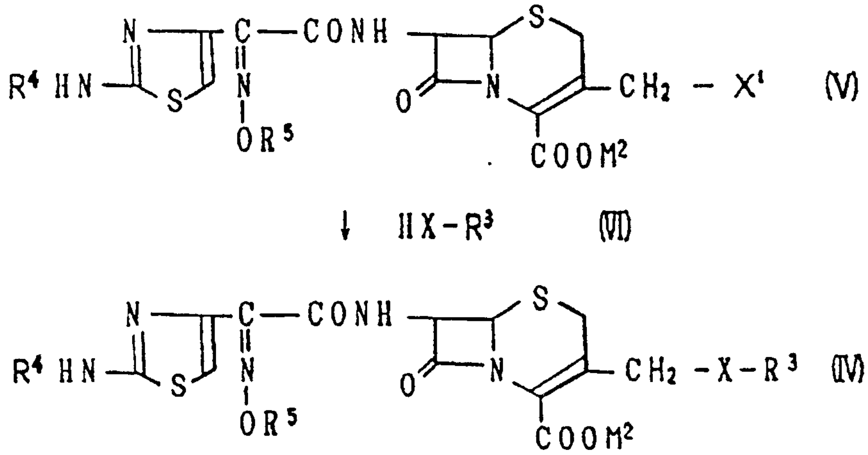


In the abovementioned formula (V), $X^1$ denotes a halogen group, $R^4$, $R^5$ and $M^2$ respectively denote the same groups as mentioned previously, and X and $R^3$ in the abovementioned formula (VI) respectively denote the same groups as mentioned previously.

A chlorine group, bromine group or iodide group is preferred as the halogen group shown by $X^1$.

The reaction between the cephem compound (V) having a halogenomethyl group in the 3-position and the hydroxy- or mercapto-pyridine derivative (VI) is preferably carried out by means of a 2 phase reaction using water and an organic solvent system; for example, it can be carried out by dissolving the compound (V) in an organic solvent, adding to this a solution in which the compound (VI) and a catalyst and base have been dissolved in water, and then vigorously stirring at 0 to 50°C, preferably at 10 to 30°C for 30 min to 5 hours.

Any of the commonly used phase transfer catalysts can be used as the abovementioned catalysts in the two-phase reaction, it being possible to mention, for example, tetrabutylammonium bromide, benzyltrimethylammonium bromide and other such quaternary ammonium salts.

Sodium carbonate, sodium hydrogen carbonate, potassium carbonate, calcium hydroxide and the like are used as the abovementioned base.

Any solvent can be used as the abovementioned organic solvent provided it is sparingly soluble in water and able to dissolve the cephem compound (V) and is a solvent which does not impose any hindrance upon the reaction, it being possible to mention, for example, methylene chloride, ethyl acetate and the like.

Moreover, there will be cases in which 2-cephem compounds are produced as by-products in the reaction between compound (V) and compound (VI) and in such cases it will either be necessary to isolate the 2-cephem compound or to convert the 2-cephem compound by-product into the 3-cephem compound (IV) using a known method.

The compound (IV) which is obtained can be made into a cephem compound with a high antimicrobial activity by means of a protective group elimination reaction in the same way as in production method 1 as required.

Furthermore, the reaction between compound (V) and compound (VI) can, in addition to the two-phase reaction, be carried out, for example, in the presence of a base, in a suitable solvent (for example, tetrahydrofuran, dimethylformamide or the like). However, in such cases, the yield is inferior to that of the two-phase reaction.

Production method 3

As regards the compound (I) of this invention, it is possible to obtain a compound in which X is an O-bonded group and $R^3$ is

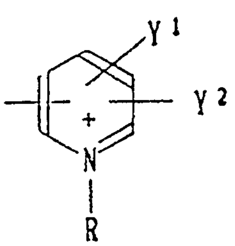

in the following way.

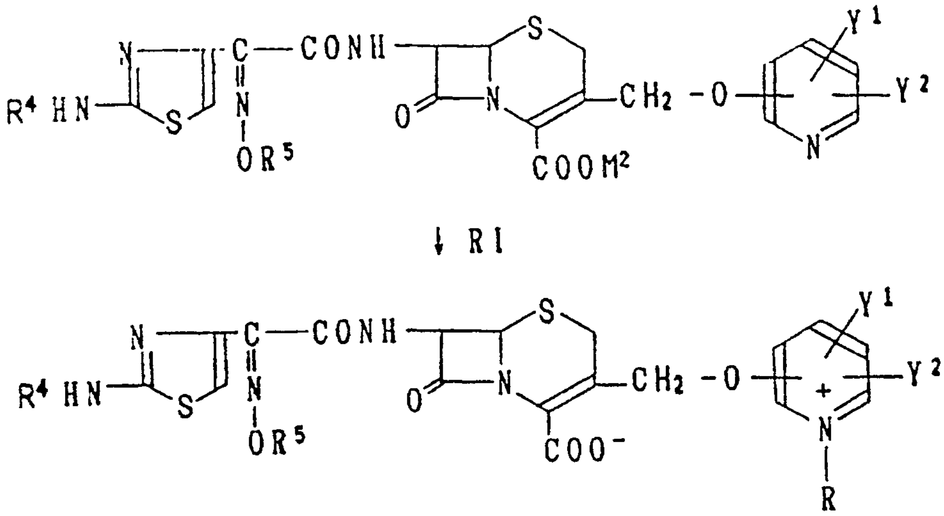


The compound which is obtained can be made into a cephem compound with a high antimicrobial activity by a protective group elimination reaction as required, in the same way as in Production Method 1.

Ethyl iodide or the like is, for example, used as the alkyl iodide (RI).

Of the target compounds (I) of this invention, pharmacologically active esters (compounds in which $M^1$ in formula (I) is a protective group providing a pharmacologically active ester) can be prepared by reacting a compound in which $M^1$ in compound (I) is hydrogen, a cation or, as $-COOM^1$, $-COO^-$ with the alcohol compounds having protective groups mentioned previously.

The cephem-1-oxide compound (III) in the above-mentioned Production Method 1 is a novel compound. This compound (III) can be obtained by reacting the 3-halogenomethylcephem 1-oxide compound (VII) and the abovementioned compound (VI) in the following way.

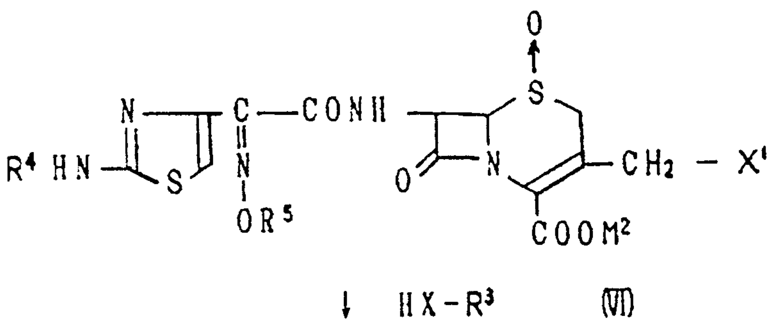

(VII)
(VI)

Compound (III)

$R^4$, $R^5$, $M^2$ and $X^1$ in the abovementioned formula (VII) denote the same groups as mentioned previously.

It is possible to carry out the reaction between compound (VII) and compound (VI) in the same way as the reaction between compound (V) and compound (VI) in Production Method 2, particularly preferably making use of a two-phase reaction.

Of the pyridine derivatives (VI) which are used as one of the starting materials in the Production Method 2 and in the production of the abovementioned intermediate compound (III), the novel compound is also included amongst the mercaptopyridine derivatives, and it is possible to obtain these in the following way.

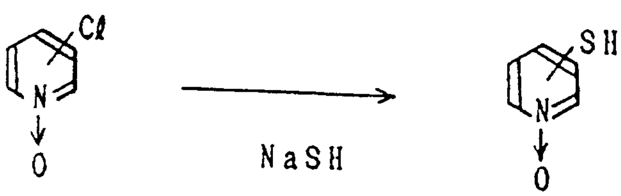


This is to say, it is possible to produce mercaptopyridine derivatives by heating chloropyridine 1-oxides with sodium hydrosulphide in a solvent such as ethyl alcohol for example.

Furthermore, when a source compound in which 2 halogen groups are present on the pyridine ring is used, 2 types of mercapto compound with the following different substitution positions are obtained.

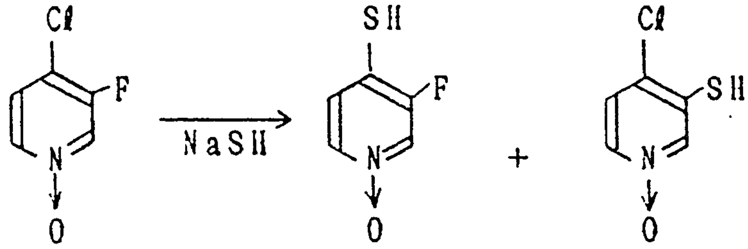


Tautomers originating from the specific behaviour of the pyridine ring are included amongst the abovementioned hydroxypyridine or mercaptopyridine derivatives (VI). For example, of the pyridine derivatives, compounds in which X in formula (VI) is an O-bonded group and $R^3$ is

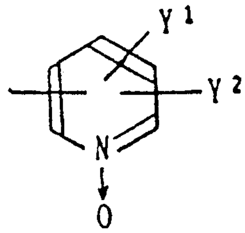

are, for example, in a state of equilibrium between the tautomers (VI a) and (VI b) shown in the following equilibrium equation. The same is true when X is a S-bonded group.

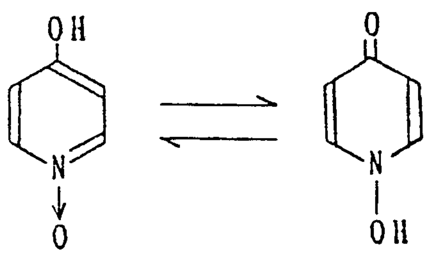

(VI a) (VI b)

This kind of tautomerism between the abovementioned pyridinol compound (VI a) and pyridone compound (VI b) is common knowledge in the relevant field and it is possible to consider such tautomers as being essentially the same compound. For the sake of convenience in this specification, it has been decided to represent the N-oxides of compound (VI) by the formula:

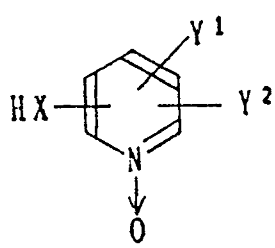

and it has been decided to adopt a nomenclature based on this formula.

In order to show the efficacy of the target compound (I) of this invention, test results for antimicrobial activity of representative compounds thereof are given.

(1) Test compounds

1. The salt of two trifluoroacetic acid molecules and 7-[(Z)-2-(2-aminothiazol-4-yl)-2-methoxyiminoacetamido]-3-(pyridin-3-yl)oxymethyl ceph-3-em-4-carboxylic acid (syn-isomer)
2. The salt of two trifluoroacetic acid molecules and 7-[(Z)-2-(2-aminothiazol-4-yl)-2-methoxyimi noacetamido]-3-(1-oxypyridin-4-yl)thiomethylceph-3-em-4-carboxylic acid (syn-isomer)

3. Sodium 7-[(Z)-2-(2-aminothiazol-4-yl)-2-(2-hydroxy-1-propyl)oxyiminoacetamido]-3-(1-oxypyridin-4-yl)thiomethylceph-3-em-4-carboxylate (syn-isomer)

4. Sodium 7-[(Z)-(2-aminothiazol-4-yl)-2-methoxyiminoacetamido]-3-(1-oxy-3-fluoropyridin-4-yl)thiomethylceph-3-em-4-carboxylate (syn-isomer)

5. Sodium 7-[(Z)-(2-aminothiazol-4-yl)-2-methoxyiminoacetamido]-3-(1-oxy-4-chloropyridin-3-yl)thiomethylceph-3-em-4-carboxylate (syn-isomer)

(2) Test results

| Compound | Minimum growth-inhibiting concentration (MIC) (μg/ml) | |
|---|---|---|
| | *1 Staphylococcus aureus 209P | *2 Escherichia coli NIHJ |
| 1 | 0 . 78 | 0 . 2 |
| 2 | 0 . 78 | 0 . 05 |
| 3 | 0 . 78 | 0 . 39 |
| 4 | 0 . 78 | 0 . 2 |
| 5 | 0 . 78 | 0 . 39 |

The above tests were carried out under culturing conditions of an inoculated bacterial amount of a bacterial growth number of $10^6$/ml, 37° C x 18 hrs., using a Bacto Mueller Hinton medium (made by the Difco Co. Ltd.) as a medium by means of the agar dish dilution method (in accordance with the standards of the Japanese Chemotherapy Society).

The target compound (I) of this invention showed a high antimicrobial activity and inhibited the growth of several pathogenic microbes including pathogenic Gram-positive bacteria and Gram-negative bacteria. According ly, this can be used as a veterinary medicine or as a medicine for the prevention or treatment of various diseases such as carbuncles, boils, scalded skin conditions, bacteraemia, abscesses of various organs, pneumonia, infectious diseases of the urinary tract, septicaemia, purulent meningitis and the like. Furthermore, it can be employed as a microbicidal agent, antifungal agent, preservative or the like.

When it is used as a medicine, it is possible to prevent or treat susceptible bacterial infections by external, local, oral or injected or suchlike administration using the usual methods, in other words by making a preparation with the addition of usual additives and administering a daily dose (adults) of 10 μg to 1 mg externally, 0.2 to 5 g intravenously, or 1 to 2 g orally, when calculated as the compound (I).

By way of the abovementioned preparation, it is possible to cite injections, internal agents, absorption agents, wet packs, eye drops, nose drops, ear drops, oral preparations, suppositories, sprays and the like as ampoules, vials, powders, pellets, granules, capsules, tablets, dry syrups, suspensions, liquids, emulsions, ointments and the like, and these are suitable for internal, external or local administration. Of the compounds (I), carboxylic acid alkali metal salts can be used in intravenous injection, droplets, intramuscular injection, intradermal injection, hypodermic injection and other such administrations, in other words as injection preparations such as ampoules, vials or the like. The pharmacologically active esters can be used in internal administrations as pellets, granules, capsules, dry syrups, liquids, tablets, suspensions and the like, and in external or localized administration applications as liquids, emulsions, ointments, suppositories, sprays and the like.

The compound (I) of this invention can also be used as a material for bacterial susceptibility regulating tests and as a source material for the synthesis of antimicrobial compounds within and outside these definitions.

The invention is now explained with the use of examples.

Example 1

[4-Methoxybenzyl (6R,7R)-7-[(Z)-2-(2-tritylaminothiazol-4-yl)-2-methoxyiminoacetamido]-3-(pyridin-3-yl)oxymethylceph-3-em-4-carboxylate 1-oxide]

1.03 g of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide was dissolved in 30 ml of acetone, 240 mg of sodium iodide were added to this and stirring was carried out for 45 min at room temperature. After concentrating under reduced pressure, stirring was carried out for 30 min with the addition of 78 mg of sodium carbonate, 4 ml of water, 145 mg of 3-hydroxypyridine, 15 ml of methylene chloride and 398 mg of tetra-n-butylammonium bromide and with ice cooling, after this vigorous stirring was carried out at room temperature for 1 hour and then extraction was carried out in ethyl acetate with the addition of ethyl acetate, ice and brine, drying was carried out with sodium sulphate after washing with water and the solvent was removed by distillation, following which 140 mg of the

target compound were obtained by developing in a column with 31 g of silica gel using a chloroform solution containing 15% by volume of methanol.
δ : 3.75(5H , b.s.) , 4.01(3H , S), 4.51(1H , d , J=4.5), 4.77, 5.23(2H , ABq , J=13) , 5.20(2H , S), 8.07(1H , dd, J=4.5 , 9), 6.66(1H , S), 6.82(2H , d , J=9), 7.0 ~ 8.5(22H , M)
IR ( $CHCl_3$) $cm^{-1}$ : 3392, 1805, 1725, 1673, 1613, 1576

Example 2

[4-Methoxybenzyl (6R,7R)-7-[(Z)-2-(2-tritylaminothiazol-4-yl)-2-methoxyiminoacetamido]-3-(pyridin-3-yl)oxymethylceph-3-em-4-carboxylate]

150 mg of the 4-methoxybenzyl (6R,7R)-7-[(Z)-2-(2-tritylaminothiazol-4-yl)-2-methoxyiminoacetamido]-3-(pyridin-3-yl)oxymethylceph-3-em-4-carboxylate 1-oxide obtained in Example 1 were dissolved in 7 ml of acetone, 172 mg of potassium iodide were added with stirring and ice cooling and following this, after the dropwise addition of 37 µl of acetyl chloride and stirring for 3 hours, extraction was carried out in ethyl acetate by the addition of ethyl acetate, water, brine and an acidic aqueous sodium sulphite solution under the action of heat, drying was carried out with sodium sulphate after washing with water and the solvent was removed by distillation, following which 78 mg of the target compound were obtained by developing the residue with ethyl acetate in a column of 57 g of silica gel.
NMR($CDCl_3$) δ : 3.57(2H , b , S), 4.74(3H , S), 4.01(3H , S), 4.83, 5.07(2H , ABq , J=14) , 4.99(1H , d , J=4.5), 5.17(2H , S), 5.88(1H , dd, J=4.5 , 9), 6.59(1H , S), 6.83(2H , d , J=9), 7.0 ~ 8.3 ( 22H , m)
IR($CHCl_3$) $cm^{-1}$ : 3407, 1787, 1717, 1702, 1612, 1585, 1570

Example 3

[(6R,7R)-7-[(Z)-2-(2-Tritylaminothiazol-4-yl)-2-methoxyiminoacetamido]-3-(pyridin-3-yl)oxymethylceph-3-em-4- carboxylic acid salt with two molecules of trifluoroacetic acid]

78 mg of the 4-methoxybenzyl (6R,7R)-7-[(Z)-2-(2-tritylaminothiazol-4-yl)-2-methoxyiminoacetamido]-3-(pyridin-3-yl)oxymethylceph-3-em-4-carboxylate obtained in Example 2 were dissolved in 1 ml of methylene chloride, 0.1 ml of anisole followed by 0.2 ml of trifluoroacetic were added dropwise with ice cooling and stirring, the solvent was removed by distillation after stirring for 1 hour, 0.35 ml of trifluoroacetic acid and 0.225 ml of water were added dropwise to the residue, again with ice cooling, at the same temperature and with stirring for 15 minutes, the solvent was removed by distillation and then 45 mg of the target compound were obtained by solidification by the addition of isopropyl ether and filtration.
NMR(DMSO-$d_6$) δ : 3.70(2H , b.s.) , 3.91(3H , S), 4.93, 5.06(2H , ABq , J=2), 5.16(1H , d , J=4.5), 5.63(1H , dd, J=4.5 , 8), 6.77(1H , s), 7.22(1H , m), 7.59(2H , m), 8.30(1H , m), 9.61(1H , d , J=8)

Example 4

[4-Methoxybenzyl (6R,7R)-7-[(Z)-2-(2-tritylaminothiazol-4-yl)-2-methoxyiminoacetamido]-3-(1-ethylpyridinium-3-yl)oxymethoxyceph-3-em-4-carboxylate iodide]

44 mg of the 4-methoxybenzyl (6R,7R)-7-[(Z)-2-(2-tritylaminothiazol-4-yl)-2-methoxyiminoacetamido]-3-(pyridin-3-yl)oxymethylceph-3-em-4-carboxylate obtained in Example 2 were dissolved in 0.7 ml of ethyl iodide and stirred for 17 hours at room temperature. The ethyl iodide was removed by distillation and 50 mg of the target compound were obtained by filtration after treating with petroleum ether.
IR (Nujol) $cm^{-1}$:
3261, 1766, 1721, 1669, 1612, 1583, 1289, 1247, 1223, 1175, 1038, 704

Example 5

[(6R,7R)-7-[(Z)-2-(2-Aminothiazol-4-yl)-2-methoxyiminoacetamido]-3-(1-ethylpyridinium-3-yl)oxymethylceph-3-em-4-carboxylate]

48 mg of the 4-methoxybenzyl (6R,7R)-7-[(Z)-2-(2-tritylaminothiazol-4-yl)-2-methoxyiminoacetamido]-3-(1-ethylpyridinium-3-yl)oxymethylceph-3-em-4-carboxylate iodide obtained in Example 4 were dissolved in 1 ml of methylene chloride, 0.1 ml of anisole followed by 0.2 ml of trifluoroacetic acid were added dropwise with ice cooling and stirring, the solvent was removed by distillation after stirring for 45 minutes, 0.35 ml of trifluoroacetic acid and 0.175 ml of water were added dropwise again with ice cooling, the solvent was removed by distillation after stirring for 2 hours at room temperature and then 21 mg of the target compound were obtained by filtration after treatment with ether, dissolving in an aqueous sodium hydrogen carbonate solution, adjusting to pH 3 and then passing through a column with a layer of 30 ml of HP20 (a polystyrene resin made by the Mitsubishi Kasei Kogyo KK) and freeze-drying.
IR (Nujol) $cm^{-1}$: 3132, 1725, 1653, 1598, 1525, 1032, 805

Example 6

[4-Methoxybenzyl (6R,7R)-7-[(Z)-2-(2-tritylaminothiazol-4-yl)-2-methoxyiminoacetamido]-3-(1-oxypyridin-3-yl)oxymethylceph-3-em-4-carboxylate 1-oxide]

500 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide were dissolved in 15 ml of acetone, 141 mg of sodium iodide were added and stirring carried out for 15 minutes with ice cooling, the solvent was removed by distillation after further stirring for 45 minutes at room temperature, 45 mg of sodium carbonate, 2 ml of water, 104 mg of 3-hydroxypyridine 1-oxide, 9 ml of methylene chloride and 150 mg of tetra-n-butylammonium bromide were added to the residue with vigorous stirring for 2 hours at room temperature and then extraction was carried out in ethyl acetate with the addition of ethyl acetate, ice, an aqueous solution of sodium hydrogen carbonate and brine, drying was carried out with sodium sulphate after washing with water and the solvent was removed by distillation, following which 130 mg of the target compound were obtained by developing the residue with ethyl acetate in a column of 60 g of silica gel.
NMR($CDC\ell_3$) $\delta$ : 3.73(3H , S), 3.92(2H , b.s.) , 4.00(3H , S), 4.70(2H , b.s.) , 5.03(1H , d , J=4.5), 5.20(2H , S), 6.07(1H , dd, J=4.5 , 9), 6.63(1H , S), 6.80(2H , d , J=8), 6.9 ~ 8.0(22H , m)
IR ($CHC\ell_3$) $cm^{-1}$ : 3397, 1804, 1724, 1677, 1612, 1585

Example 7

[4-Methoxybenzyl (6R,7R)-7-[(Z)-2-(2-tritylaminothiazol-4-yl)-2-methoxyiminoacetamido]-3-(1-oxypyridin-3-yl)oxymethylceph-3-em-4-carboxylate]

140 mg of the 4-methoxybenzyl (6R,7R)-7-[(Z)-2- (2-tritylaminothiazol-4-yl)-2-methoxyiminoacetamido]--3-(1-oxypyridin-3-yl)oxymethylceph-3-em-4-carboxylate 1-oxide obtained in example 6 were dissolved in 6 ml of acetone, 170 mg of potassium iodide followed by 37 µl of acetyl chloride were added with ice cooling and stirring and stirring was carried out for 1 hour and 40 minutes, extraction was carried out in ethyl acetate with the addition of ethyl acetate, ice and an acidic aqueous sodium sulphite solution, drying was carried out with sodium sulphate after washing with water and the solvent was removed by distillation: 80 mg of the target compound were obtained by developing the residue with ethyl acetate in a column of 57 g of silica gel.
NMR($CDC\ell_3$) $\delta$ : 3.77(3H , S), 3.90(2H , b.s.) , 4.00(3H , S), 4.65, 4.95(2H , ABq , J=14) , 5.05(1H , d , J=4.5), 5.93(1H , dd, J=4.5 , 9), 6.83(1H , S), 6.85(2H , d , J=8), 6.9 ~ 8.0(22H, m)
IR ($CHC\ell_3$) $cm^{-1}$ : 3401, 1795, 1718, 1687, 1614, 1587

Example 8

[(6R,7R)-7-[(Z)-2-(2-Aminothiazol-4-yl)-2-methoxyiminoacetamido]-3-(1-oxypyridin-3-yl)oxymethylceph-3-em-4-carboxylic acid salt with two molecules of trifluoroacetic acid]

80 mg of the 4-methoxybenzyl (6R,7R)-7-[(Z)-2-(2-tritylaminothiazol-4-yl)-2-methoxyiminoacetamido]-3-(1-oxypyridin-3-yl)oxymethylceph-3-em-4-carboxylate obtained in Example 7 were dissolved in 1 ml of methylene chloride, 0.1 ml of anisole and 0.2 ml of trifluoroacetic acid were added with ice cooling and stirring, and, after stirring for 45 minutes, stirring was carried out for 15 minutes at room temperature, the solvent war removed by distillation and then 0.4 ml of trifluoroacetic acid and 0.15 ml of water were added with stirring for 1.5 hours at room temperature again with ice cooling, the solvent was removed by distillation and then 35 mg of the target compound were obtained by solidifying by the addition of ether and filtration.
NMR(DMSO-$d_6$) $\delta$ : 3.85(3H , S), 3.79(2H , b.s.) , 5.01(1H , d , J=4.5), 4.80, 5.15(2H , ABq , J=14) , 5.83(1H , dd, J=4.5 , 9), 8.73(1H , S), 6.7 ~ 8.2(4H,m)
IR (Nujol)) $cm^{-1}$:
3285, 1786, 1666, 1202, 1136, 1142

Example 9

[4-Methoxybenzyl (6R,7R)-7-[(Z)-2-(2-tritylaminothiazol-4-yl)-2-methoxyiminoacetamido]-3-(4-oxo-1,4-dihydropyridin-3-yl)oxymethylceph-3-em-4-carboxylate 1-oxide]

500 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide were dissolved in 15 ml of acetone, 120 mg of sodium iodide were added with stirring for 1.5 hours at room temperature, the solvent was removed by distillation, 40 mg of sodium carbonate, 2 ml of water, 137 mg of 4-hydroxypyridine 1-oxide, 7.8 ml of methylene chloride and 120 mg of tetra-n-butylammonium bromide were added and stirring carried out for 2.5 hours at room temperature, following which the solvent was removed by distillation, extraction was carried out in ethyl acetate, drying was carried out with sodium sulphate after washing with water and the solvent was removed by distillation, following which 370 mg of the target compound were obtained by developing the residue in a column with a

layer of 80 g of silica gel, using ethyl acetate containing a 10% by volume methanol.
NMR($CDC\ell_3$) δ : 3.73(3H , S), 3.99(3H , S), 6.63(1H , S), 6.74 ~7.67 (21H, m)
IR(KBr) $cm^{-1}$ : 3351, 2924, 1804, 1725, 1686, 1512, 1340, 1306, 1250, 1174, 1036, 822 , 753 , 700

Example 10

[4-Methoxybenzyl
(6R,7R)-7-[(Z)-2-(2-tritylaminothiazol-4-yl)-2-methoxyiminoacetamido]-3-(4-oxo-1,4-dihydropyridin-1-yl)oxymethylceph-3-em-4-carboxylate]

370 mg of the 4-methoxybenzyl (6R,7R)-7-[(Z)-2-(2-tritylaminothiazol-4-yl)-2-methoxyiminoacetamido]--3-(4-oxo-1,4-dihydroxypyridin-1-yl)oxymethylceph-3-em-4-carboxylate 1-oxide obtained in Example 9 were dissolved in 18.5 ml of acetone, 426 mg of potassium iodide were added, stirring was carried out for 30 min at -10°C, following which 0.93 ml of acetyl chloride were added, stirring was carried out for 30 min at the same temperature, and an acidic aqueous sodium sulphite solution was added, extraction was carried out in ethyl acetate, drying was carried out with sodium sulphate after washing in water and the solvent removed by distillation: 100 mg of the target compound were obtained by developing the residue with a mixed solvent of benzene and ethyl acetate (3:1 volume ratio) in a column with a layer of 50 g of silica gel.
NMR($CDC\ell_3$) δ : 3.37 (1H , d , J=19) , 3.77(3H , S), 4.00(1H , d , J=19 ) , 4.04(3H , S), 5.02(1H , d , J=5.6), 5.26(2H , S), 5.99(1H , dd, J=5.6 , 9.0), 6.59(1H , S), 6.77~7.71(21H, m).

Example 11

[(6R,7R)-7-[(Z)-2-(2-Aminothiazol-4-yl)-2-methoxyiminoacetamido]-3-(4-oxo-1,4-dihydropyridin-1-yl)oxymethylceph-3-em-4-carboxylic acid, trifluoroacetic acid salt]

100 mg of the 4-methoxybenzyl (6R,7R)-7-[(Z)-2-(2-tritylaminothiazol-4-yl)-2-methoxyiminoacetamido]--3-(4-oxopyridin-1-yl)oxymethylceph-3-em-4-carboxylate obtained in Example 10 were dissolved in 0.83 ml of methylene chloride, concentration was carried out after adding 0.1 ml of anisole and 0.16 ml of trifluoroacetic acid with ice cooling and stirring and stirring for 30 minutes at the same temperature and for a further 30 minutes at room temperature, the solvent was removed by distillation after adding 0.47 ml of trifluoroacetic acid and 0.23 ml of water again with ice cooling and stirring for 30 minutes at the same temperature and for a further 40 minutes at room temperature: 32 mg of the target compound were obtained by solidifying by adding ethyl ether and filtering.
NMR(DMSO-$d_6$) δ : 3.71(2H , b.s.) , 3.91(3H , S), 5.21(1H , d , J=5), 5.98(1H , dd, J=5.8), 6.84(1H , S), 7.24(2H , S), 7.31(2H , S), 8.02(3H , b.s.) , 9.74(1H , d , J=8)
IR(KBr) $cm^{-1}$ : 3274, 1788, 1671, 1540, 1431, 1306, 1204, 1137, 1045, 1007, 801 , 724

Example 12

[4-Methoxybenzyl
(6R,7R)-7-[(Z)-2-(2-tritylaminothiazol-4-yl)-2-methoxyiminoacetamido]-3-(1-oxypyridin-4-yl)thiomethylceph-3-em-4-carboxylate 1-oxide]

500 mg of 4-methoxybenzyl (6R,7R)-7-[(Z)-2-(2-tritylaminothiazol-4-yl)-2-methoxyiminoacetamido]-3-chloromethylceph-3-em-4-carboxylate were dissolved in 15 ml of acetone, 121 mg of sodium iodide were added with stirring for 1.5 hours at room temperature, following which the solvent was removed by distillation, the residue was ice cooled, 40 mg of sodium carbonate, 2 ml of water, 157 mg of 4-mercaptopyridine 1-oxide, 120 mg of tetra-n-butylammonium bromide and 8 ml of methylene chloride were added with stirring for 1 hour at the same temperature and for a further 2 hours at room temperature, following which 10 ml of water were added, extraction was carried out with chloroform, drying was carried out with sodium sulphate after washing with water and the solvent removed by distillation: 310 mg of the target compound were obtained by developing the residue with chloroform in a column with a layer of 37 g of silica gel.
NMR($CDC\ell_3$) δ : 3.31, 4.11(2H , ABq , J=18.8) , 3.77(3H , S), 4.02(3H , S), 4.59(1H , d , J=4.8 ) , 5.11(2H , S), 6.01(1H , dd, J=4.8 , 10) , 6.67(1H , S), 6.77~8.13(21H, m)
IR(KBr) $cm^{-1}$ : 3374, 2939, 1798, 1725, 1680, 1617, 1520, 1470, 1302, 1250, 1177, 1037, 830 , 755 , 706 , 666

Example 13

[4-Methoxybenzyl
(6R,7R)-7-[(Z)-2-(2-tritylaminothiazol-4-yl)-2-methoxyiminoacetamido]-3-(1-oxypyridin-4-yl)thiomethylceph-3-em-4-carboxylate]

310 mg of the 4-methoxybenzyl (6R,7R)-7-[(Z)-2- (2-tritylaminothiazol-4-yl)-2-methoxyiminoacetamido]--3-(1-oxypyridin-4-yl)thiomethylceph-3-em-4-carboxylate 1-oxide obtained in Example 12 were dissolved in 15.5 ml of acetone, 457 mg of potassium iodide were added and stirring carried out for 30 minutes cooling to -20°C, following which 0.78 ml of acetyl chloride were added and stirring carried out for 30 minutes at the same temperature and for a further 30 minutes at -8 to -5°C, after which the reaction liquid was added to an acidic

aqueous sodium sulphite solution, extraction was carried out in ethyl acetate, drying was carried out with sodium sulphate after washing with water and the solvent was removed by distillation: 240 mg of the target compound were obtained by developing the residue in a chloroform solution containing 10% by volume of methanol in a column with a layer of 18 g of silica gel.
NMR($CDCl_3$) δ : 3.50(2H , b.s.) , 3.76(3H , S), 3.95(3H , S), 4.05(2H , b.s.) , 4.98(1H , d , J=4.4), 5.12(2H , S), 5.81(1H , dd, J=4.4 , 8), 6.56(1H , S), 6.77~7.34(18H, m), 7.92(2H , d , J=7), 8.39(1H , d , J=8)
IR(KBr) $cm^{-1}$ : 3374, 2939, 1783, 1718, 1677, 1614, 1515, 1467, 1363, 1301, 1242, 1175, 1036, 907 , 867 , 754 , 704 , 666

Example 14

[(6R,7R)-7-[(Z)-2-(2-Aminothiazol-4-yl)-2-methoxyiminoacetamido]-3-(1-oxypyridin-4-yl)thiomethylceph-3-em-4-carboxylic acid, trifluoroacetic acid salt]

220 mg of the 4-methoxybenzyl (6R,7R)-7-[(Z)-2-(2-tritylaminothiazol-4-yl)-2-methoxyiminoacetamido]-3-(1-oxypyridin-4-yl)thiomethylceph-3-em-4-carboxylate obtained in Example 13 were dissolved in 2 ml of methylene chloride, this was cooled to -6°C and then 0.22 ml of anisole and 0.36 ml of trifluoroacetic acid were added with stirring for 30 minutes at the same temperature and for a further 2 hours at room temperature, the solvent was removed by distillation and then 142 mg of the target compound were obtained by solidification by the addition of petroleum benzyl and ethyl ether and filtration.
NMR(DMSO-$d_6$ ) δ : 3.74(2H , b.s.) , 3.92(3H , S), 4.26(2H , b.s.) , 5.20(1H , d , J=4.8), 5.78(1H , dd, J=4.8 , 7.6), 6.87(1H , S), 7.05~7.63(5H , m), 8.35(2H , d , J=6.8), 9.69(1H , d , J=7.6)
IR(KBr) $cm^{-1}$ : 3062, 1770, 1662, 1176, 1137, 1043, 835 , 799 , 721 , 661

Example 15

[4-Methoxybenzyl (6R,7R)-7-[(Z)-2-(2-tritylaminothiazol-4-yl)-2-methoxyiminoacetamido]-3-(1-oxypyridin-3-yl)thiomethylceph-3-em-4-carboxylate 1-oxide]

A reaction and treatment were carried out in the same way as in Example 9 using 500 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide and 160 mg of 3-mercaptopyridine 1-oxide to obtain 257 mg of the target compound.
NMR($CDCl_3$) δ : 3.79(3H , S), 4.47(1H , d , J=15.4) , 4.94(1H , d , J=5.0), 5.16(2H , S), 5.53(1H , S), 5.98(1H , dd, J=5.0 , 7.4), 6.55(1H , S), 6.71~7.61(20H, m), 7.71~8.81(4H , m), 9.83(1H , d , J=7.4)
IR(KBr) $cm^{-1}$ : 3377, 2927, 1792, 1725, 1670, 1611, 1585, 1521, 1467, 1414, 1291, 1239, 1161, 1096, 1035, 1012, 912 , 752 , 700 , 677 , 551

Example 16

[4-Methoxybenzyl (6R,7R)-7-[(Z)-2-(2-tritylaminothiazol-4-yl)-2-methoxyiminoacetamido]-3-(oxypyridin-3-yl)thiomethylceph-3-em-4-carboxylate]

219 mg of the 4-methoxybenzyl (6R,7R)-7-[(Z)-2-(2-tritylaminothiazol-4-yl)-2-methoxyiminoacetamido]--3-(1-oxypyridin-3-yl)thiomethylceph-3-em-4-carboxylate 1-oxide obtained in Example 15 were dissolved in 11 ml of acetone, 332 mg of potassium iodide and 0.57 ml of acetyl chloride were added to this, following which the reaction and treatment were carried out in the same way as in Example 13 to obtain 163 mg of the target compound.
NMR($CDCl_3$) δ : 3.54(2H , b.s.) , 3.79(3H , S), 4.02(3H, S), 5.01(1H , d , J=4.8), 5.11(2H , S), 5.86(1H , dd, J=4.8 , 8.7), 6.65(1H , S), 6.76~7.66(22H, m), 7.66~8.36(3H , m)
IR(KBr) $cm^{-1}$ : 3403, 2934, 1781, 1717, 1676, 1610, 1583, 1512, 1489, 1466, 1446, 1413, 1357, 1296, 1243, 1172, 1159, 1049, 1035, 1014, 910 , 754 , 702

Example 17

[Sodium (6R,7R)-7-[Z)-2-(2-aminothiazol-4-yl-2-methoxyiminoacetamido]-3-(1-oxypyridin-3-yl)thiomethylceph-3-em 4-carboxylate]

80 mg of the 4-methoxybenzyl (6R,7R)-7-[(Z)-2-(2-tritylaminothiazol-4-yl)-2-methoxyiminoacetamido]-3-(oxypyridin-3-yl)thiomethylceph-3-em-4-carboxylate obtained in Example 16 were dissolved in 0.7 ml of methylene chloride, this was cooled to -5°C, 0.08 ml of anisole and 0.13 ml of trifluoroacetic acid were added dropwise with stirring, stirring was carried out for 30 minutes at the same temperature and for a further 1 hour at room temperature, following which the solvent was removed by distillation, the residue was stirred for 2 hours at room temperature with the dropwise addition of 0.37 ml of trifluoroacetic acid followed by 0.19 ml of water again with ice cooling, following which the solvent was removed by distillation and 47 mg of the trifluoroacetic acid salt of the target compound were obtained by treating with n-hexane and ether. This substance was dissolved in an aqueous solution of sodium hydrogen carbonate, purified by passing through a

column of 50 ml of HP20 and then freeze dried to obtain the target compound.
NMR(DMSO-d6) δ : 3.84(3H , S), 4.15(1H , d , J=13.2) , 4.54(1H, d , J=13.2) , 4.98(1H, d , J=4.4), 5.57(1H , dd, J=4.4 , 7.6), 6.74(1H , S), 7.02~7.67(4H , m), 7.90~8.47(2H , m), 9.52(1H , d , J=7.6)
IR(KBr) $cm^{-1}$ : 3415, 1757, 1665, 1606, 1531, 1471, 1415, 1386, 1355, 1285, 1224, 1178, 1101, 1039, 910 , 793 , 669 , 554

Example 18

[4-methoxybenzyl (6R,7R)-7-[(Z)-2-(2-tritylaminothiazol-4-yl)-2-(2-trimethylsilyloxy-1-propyl)oxyiminoacetamido]-3-(1-oxypyridin-4-yl)thiomethylceph-3-em-4-carboxylate 1-oxide]

427 mg of 4-methoxybenzyl (6R,7R)-7-[(Z)-2-(2-tritylaminothiazol-4-yl)-2-(2-trimethylsilyloxy-1-propyl)oxyiminoacetamido]-3-chloromethylceph-3-em-4-carboxylate-1-oxide were dissolved in 15 ml of acetone, 114 mg of sodium iodide were added, stirring was carried out for 45 minutes at room temperature, following which extraction was carried out in ethyl acetate, drying was carried out with sodium sulphate after washing with water, the solvent was removed by distillation, the residue was again cooled, 44 mg of sodium carbonate, 2 ml of water, 117 mg of 4-mercaptopyridine 1-oxide, 8 ml of methylene chloride followed by 124 mg of tetra-n-butylammonium bromide were added, vigorous stirring was carried out for 45 minutes at room temperature and then this was dissolved in ethyl acetate; washing was carried out with an aqueous solution of sodium hydrogen carbonate, drying was carried out with sodium sulphate and the solvent was removed by distillation, following which 302 mg of the target compound were obtained by developing the residue in a column with a layer of 30 g of silica gel using a mixed solution of ethyl acetate and acetone (1:4 volume ratio).
NMR(CDCℓ3) δ : 0.85(9H , S), 1.13(3H , d , J=4), 3.32(1H , d , J=18) , 3.75(3H , S), 4.08(5H , b.s.) , 4.53(1H , d , J=4.5), 5.07(2H , S), 6.00(1H , dd, J=4.5 , 9), 6.63(1H , S), 6.83(2H , d , J=9), 7.0 ~7.7(20H , m), 7.86(2H , d , J=6.5)
IR(CHCℓ3) $cm^{-1}$ : 3397, 1802, 1722, 1700, 1612, 1585, 1512, 1035, 929

Example 19

[4-Methoxybenzyl (6R,7R)-7-[(Z)-2-(2-tritylaminothiazol-4-yl)-2-(2-trimethylsilyloxy-1-propyl)oxyiminoacetamido]-3-(1-oxypyridin-4-yl)thiomethylceph-3-em-4-carboxylate]

302 mg of the 4-methoxybenzyl (6R,7R)-7-[(Z)-2-(2-tritylaminothiazol-4-yl)-2-(2-trimethylsilyloxy-1-propyl)oxyiminoacetamido]-3-(1-oxypyridin-4-yl)thiomethylceph-3-em-4-carboxylate 1-oxide obtained in Example 18 were dissolved in 8.5 ml of acetone, 296 mg of potassium iodide followed by 63 µl of acetyl chloride were added with ice cooling and stirring, and then 256 mg of the target compound were obtained by reacting and treating in the same way as in Example 13.
NMR(CDCℓ3) δ : 0.90(9H , S), 1.16(3H , d , J=4), 3.51(2H , t , J=18) , 3.80(3H , S), 4.10(5H , b.s.) , 4.99(1H , d , J=4.5), 5.16(2H , S), 5.83(1H , dd, J=4.5 , 9), 6.70(1H , S), 6.88(2H , d , J=9), 6.9 ~7.6(19H , m), 7.83(1H , d , J=9), 7.95(2H , d , J=6.5)
IR(CHCℓ3) $cm^{-1}$ : 3413, 1787, 1718, 1611, 1584, 1513, 1035, 929

Example 20

[Sodium (6R,7R)-7-[(Z)-2-(2-aminothiazol-4-yl)-2-(2-hydroxy-1-propyl)oxyiminoacetamido]-3-(1-oxypyridin-4-yl)thiomethylceph-3-em-4-carboxylate]

256 mg of the 4-methoxybenzyl (6R,7R)-7-[(Z)-2-(2-tritylaminothiazol-4-yl)-2-(2-trimethylsilyloxy-1-propyl)oxyiminoacetamido]-3-(1-oxypyridin-4-yl)thiomethylceph-3-em-4-carboxylate obtained in Example 19 were used to carry out reaction and treatment in the same way as in Example 17 and 50 mg of the target compound were obtained.
NMR(DMSO-d6) δ : 1.07(3H , d , J=4), 3.92(3H , b.s.) , 4.28, 4.48(2H , ABq , J=14) , 4.99(1H, d , J=4.5), 5.58(1H, dd, J=4.5, 9), 6.70(1H, S), 7.23(2H, b.s.) , 7.47 , 8.02(4H, ABq , J=6.5), 9.48(1H , d , J=9)
IR (Nujol) $cm^{-1}$: 3290, 1761, 1661, 1595, 1532, 1212,

Example 21

[4-Methoxybenzyl (6R,7R)-7-[(Z)-2-(2-tritylaminothiazol-4-yl)-2-methoxyiminoacetamido]-3-(1-oxy-4-chloropyridin-3-yl)thiomethylceph-3-em-4-carboxylate 1-oxide]

(1) 883 mg of 3-fluoro-4-chloropyridine 1-oxide [NMR($CDCl_3$) δ: 7.35 (1H, dd, J=7,8), 8.07 (1H, dd, J=1.5, 7), 8.27 (1H, dd, J=1.5, 4.5)] were dissolved in 60 ml of 99% by volume of ethanol, 960 mg of sodium hydrosulphide were added and heating reflux carried out for 1.5 hours at 70 to 80°C. Impurities were removed by filtration, the solvent was removed by distillation, and 265 mg of 4-chloro-3-mercaptopyridine 1-oxide and 468 mg of 3-fluoro-4-mercaptopyridine 1-oxide were obtained by treating the residue with ether.

[4-Chloro-mercaptopyridine 1-oxide]

NMR(DMSO-$d_6$) δ : 3.57(1H, b.s.), 6.99(1H, d, J=7), 7.40(1H, dd, J=2,7), 8.11(1H, d, J=2)

[3-Fluoro-4-mercaptopyridine 1-oxide]

NMR(DMSO-$d_6$) δ : 3.57(1H, b.s.), 7.10(1H, t, J=7), 7.43(1H, dd, J=2.7), 7.88(1H, dd, J=2, 4.5)

(2) 218 mg of the target compound were obtained by carrying out reaction and treatment in the same way as in Example 18 using 500 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide and 198 mg of the 3-mercapto-4-chloropyridine 1-oxide obtained in the abovementioned (1).

NMR($CDCL_3$) δ : 3.76(5H, b.s.), 4.02(5H, b.s.), 4.59(1H, d, J=4.5), 5.16(2H, S), 6.02(1H, dd, J=4.5, 9), 6.66(1H, S), 6.84(2H, d, J=9), 7.0~7.7(19H, m), 7.83(1H, dd, J=1.5, 7), 8.12(1H, d, J=1.5)

IR($CHCl_3$) $cm^{-1}$ : 3405, 1802, 1724, 1685, 1614, 1588, 1516, 1049, 928

Example 22

[4-Methoxybenzyl (6R,7R)-7-[(Z)-2-(2-tritylaminothiazol-4-yl)-2-methoxyiminoacetamido]-3-(1-oxy-3-fluoropyridin-4-yl)thiomethylceph-3-em-4-carboxylate 1-oxide]

233 mg of the target compound were obtained by reaction and treatment in the same way as in Example 18 using 500 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide and 179 mg of the 3-fluoro-4-mercaptopyridine 1-oxide obtained in the abovementioned Example 21, (1).

NMR($CDCl_3$) δ : 3.34, 4.12(2H, ABq, J=18), 3.79(3H, S), 4.05(5H, b.s.), 4.55(1H, d, J=4.5), 5.07(2H, S), 6.03(1H, dd, J=4.5, 9), 6.65(1H, S), 6.85(2H, d, J=9), 7.0~7.7(18H, m), 7.73(1H, b.s.), 7.80(1H, dd, J=1.5, 6), 7.98(1H, dd, J=1.5, 4.5)

Example 23

[4-Methoxybenzyl (6R,7R)-7-[(Z)-2-(2-tritylaminothiazol-4-yl)-2-methoxyiminoacetamido]-3-(1-oxy-4-chloropyridin-3-yl)thiomethylceph-3-em-4-carboxylate]

218 mg of the 4-methoxybenzyl (6R,7R)-7-[(Z)-2-(2-tritylaminothiazol-4-yl)-2-methoxyiminoacetamido]--3-(1-oxy-4-chloropyridin-3-yl)thiomethylceph-3-em-4-carboxylate-1-oxide obtained in Example 21 were dissolved in 9 ml of acetone, 219 mg of potassium iodide and 47 μl of acetyl chloride were added with ice cooling and stirring, following which 204 mg of the target compound were obtained by reaction and treatment in the same way as in Example 13.

NMR($CDCl_3$) δ : 3.58(2H, b.s.), 3.80(3H, S), 4.04(5H, b.s.), 5.04(1H, d, J=4.5), 5.13(2H, S), 5.88(1H, dd, J=4.5, 9), 6.69(1H, S), 6.87(2H, d, J=9), 7.0 ~ 7.7(19H, m), 7.90(1H, dd, J=2.7), 8.18(1H, d, J=2)

IR ($CHCl_3$) $cm^{-1}$ : 3410, 1788, 1721, 1679, 1611, 1586, 1517, 1043, 929

Example 24

[Sodium (6R,7R)-7-[(Z)-2-(2-aminothiazol-4-yl)-2-methoxyiminoacetamido]-3-(1-oxy-4-chloropyridin-3-yl)thiomethylceph-3-em-4-carboxylate]

28 mg of the target compound were obtained by reaction and treatment in the same way as in Example 17 using 204 mg of the 4-methoxybenzyl (6R,7R)-7-[(Z)-2-(2-tritylaminothiazol-4-yl)-2-methoxyiminoacetamido]-3-(1-oxy-4-chloropyridin-3-yl)thiomethylceph-3-em-4-carboxylate obtained in Example 23.

NMR(DMSO-$d_6$) δ : 3.45(2H, b.s.), 3.85(3H, b.s.), 4.37(2H, b.s.), 5.02(1H, d, J=4.5), 5.57(1H, dd, J=4.5, 9), 6.71(1H, S), 7.15(2H, b.s.), 7.45(1H, d, J=6), 7.99(1H, dd, J=2.6), 8.58(1H, d, J=2), 9.48(1H, d, J=9)

IR(Nujol) $cm^{-1}$: 3357, 1760, 1663, 1602, 1533, 1040

Example 25

[4-Methoxybenzyl (6R,7R)-7-[(Z)-2-(2-tritylaminothiazol-4-yl)-2-methoxyiminoacetamido]-3-(1-oxy-3-fluoropyridin-4-yl)thiomethylceph-3-em-4-carboxylate]

233 mg of the 4-methoxybenzyl (6R,7R)-7-[(Z)-2-(2-tritylaminothiazol-4-yl)-2-methoxyiminoacetamido]--3-(1-oxy-3-fluoropyridin-4-yl)thiomethylceph-3-em-4-carboxylate 1-oxide obtained in Example 22 were dissolved in 10 ml of acetone, 253 mg of potassium iodide and 54 μl of acetyl chloride were added, after which 192 mg of the target compound were obtained by reaction and treatment in the same way as in Example 13.

NMR($CDCl_3$) δ : 3.55(2H, b.s.), 3.80(3H, S), 4.02(5H, b.s.), 4.99(1H, d, J=4.5), 5.10(2H, S), 5.84(1H, dd, J=4.5, 9), 6.63(1H, S), 6.88(2H, d, J=9), 6.8~ 7.4(19H, m), 7.65(1H, d, J=9), 7.87(1H, dd, J=1.5, 7), 8.01(1H, dd, J=1.5, 4.5)

IR($CHCl_3$) $cm^{-1}$ : 3422, 1789, 1720, 1686, 1614, 1587, 1518, 1042, 930

Example 26

[Sodium (6R,7R)-7-[(Z)-2-(2-aminothiazol-4-yl)-2-methoxyiminoacetamido]-3-(1-oxy-4-fluoropyridin-4-yl)thiomethylceph-3-em-4-carboxylate]

25 mg of the target compound were obtained by reaction and treatment in the same way as in Example 17 using 192 mg of the 4-methoxybenzyl (6R,7R)-7-[(Z)-2-(2-tritylaminothiazol-4-yl)-2-methoxyiminoacetamido]-3-(1-oxy-3-fluoropyridin-4-yl)thiomethylceph-3-em-4-carboxylate obtained in Example 25.
NMR(DMSO-$d_6$) δ : 3.45(2H, b.s.), 3.85(3H, S), 4.25, 4.35(2H, ABq, J=14), 4.98(1H, d, J=4.5), 5.57(1H, dd, J=4.5, 9), 6.72(1H, S), 7.16(2H, b.s.), 7.7~8.2(2H, m), 8.36(1H, dd, J= 1.5, 5), 9.50(1H, d, J=9)
IR (Nujol) $cm^{-1}$: 3305, 1758, 1663, 1601, 1532, 1284, 1165, 1037

Example 27

[4-Methoxybenzyl (6R,7R)-7-[(Z)-2-(2-tritylaminothiazol-4-yl)-2-methoxyiminoacetamido]-3-(1-oxypyridin-4-yl)thiomethylceph-3-em-4-carboxylate]

6.5 mg of sodium carbonate, 0.33 ml of water, 19.5 mg of tetra-n-butylammonium bromide and 26 mg of 4-mercaptopyridine 1-oxide were added to a solution in which 88 mg of 4-methoxybenzyl (6R,7R)-7-[(Z)-2-(2-tritylaminothiazol-4-yl)-2-methoxyiminoacetamido]-3-(iodomethylceph-3-em-4-carboxylate [ NMR($CDCl_3$) δ : 3.44(1H, d, J=18.4), 3.69(1H, d, J=18.4), 3.75(3H, S), 4.05(3H, S), 4.33(2H, S), 4.95(1H, d, J=4.6), 5.18(2H, S), 5.77(1H, dd, J=4.6, 8.6), 6.52(1H, S), 6.73 ~ 7.64(21H, m) ; IR(KBr) $cm^{-1}$ : 3408, 2950, 1791, 1725, 1684, 1619, 1521, 1453, 1390, 1366, 1308, 1251, 1186, 1045, 832, 764, 711] had been dissolved in 1.3 ml of methylene chloride with stirring for 1 hour; after further stirring for 2 hours at room temperature, extraction was carried out with the addition of chloroform, drying was carried out with sodium sulphate after washing with water, the solvent was removed by distillation and 33 mg of the target product were obtained by developing the residue in a column with a layer of 25 g of silica gel, using chloroform containing 10% by volume of methanol. This substance was an identical substance to the compound obtained in Example 13.

In addition to this compound, 25 mg of 4-methoxybenzyl (6R,7R)-7-[(Z)-2-(2-tritylaminothiazol-4-yl)-2-methoxyiminoacetamido]-3-(1-oxypyridin-4-yl)thiomethylceph-2-em-4-carboxylate were obtained.
NMR($CDCl_3$) δ : 3.70(2H, b.s.), 3.81(3H, S), 3.94(3H, S), 5.14(2H, S), 5.27 (1H, S), 5.27 (1H, d, J=4.0), 5.71(1H, dd, J=4.0, 9.0), 6.14(1H, S), 6.62(1H, S), 6.80 ~ 8.36(25H, m)
IR(KBr)$cm^{-1}$ : 3407, 2931, 1777, 1738, 1675, 1612, 1514, 1491, 1466, 1445, 1384, 1247, 1175, 1035, 844, 828, 753, 702

Example 28

[4-Methoxybenzyl (6R,7R)-7-[(Z)-2-(2-tritylaminothiazol-4-yl)-2-methoxyiminoacetamido]-3-(1-oxypyridin-4-yl)thiomethylceph-3-em-4-carboxylate-1-oxide]

32 mg of 4-mercaptopyridine 1-oxide were added to 3 ml of dimethylformamide with ice cooling and stirring, 15 mg of potassium carbonate were added with stirring for 30 minutes at room temperature, following which 100 mg of 4-methoxybenzyl (6R,7R)-7-[(Z)-2-(2-tritylaminothiazol-4-yl)-2-methoxyiminoacetamido]-3-chloromethylceph-3-em-4-carboxylate 1-oxide were added, stirring was carried out for 2 hours at room temperature and then the solvent was removed by distillation, extraction was carried out with the addition of chloroform to the residue, drying was carried out with sodium sulphate after washing with water, the solvent was removed by distillation and 30 mg of the target compound were obtained by developing the residue in a column with a layer of 30 g of silica gel, using chloroform containing 10% by volume of methanol. This substance was identical to the compound obtained in Example 12.

Example 29

[Pivaloyloxymethyl (6R,7R)-7-[(Z)-2-(2-aminothiazol-4-yl)-2-methoxyiminoacetamido]-3-(1-oxy-3-fluoropyridin-4-yl)thiomethylceph-3-em-4-carboxylate]

15 µl of chloromethyl pivalate were dissolved in 3 ml of acetone, stirring was carried out for 1 hour at room temperature after which the solvent was removed by distillation, the residue was dissolved in 0.7 ml of dimethylformamide, 22 mg of sodium carbonate and 44 mg of sodium (6R,7R)-7-[(Z)-2-(2-aminothiazol-4-yl)-2-methoxyiminoacetamido]-3-(1-oxy-3-fluoropyridin-4-yl)thiomethylceph-3-em-4-carboxylate (obtained in Example 26) were added to the solution with ice cooling and stirring, stirring for 30 minutes, with further stirring for 30 minutes at room temperature, extraction was carried out with the addition of ethyl acetate, drying was carried out with sodium sulphate after washing with water, the solvent was removed by distillation: 15 mg

of the target compound were obtained by developing the residue in a column with a layer of 10 g of silica gel using chloroform containing 5% by volume of methanol.
IR($CHCl_3$) $cm^{-1}$ : 3473, 3398, 1781, 1751, 1655, 1608, 1112, 1035, 931

Advantages of the invention

The compound of this invention exhibits a high antimicrobial activity and is useful as a preventative or therapeutic medicine for various susceptible bacterial infections.

Example 30

Preparation of sodium (6R,7R)-7-[(Z)-2-(2-aminothiazol-4-yl)-2-methoxyiminoacetamido]-3-(1-oxypyridin-4-yl)thiomethylceph-3-em-4-carboxylate.

Trifluoroacetate, obtained in Example 14, was dissolved in 5% $NaHCo_3$ to adjust the pH to 7.5, and subjected to column chromatography on a non-ionic adsorption resin (Diaion HP-20). The column was washed with $H_2O$ and eluted with 15% aqueous methyl alcohol, followed bv lyophilization of fractions containing the desired product to afford the titled compound.
$IR_\nu KBr$ max $cm^{-1}$: 3396, 1761, 1663, 1601, 1531, 1469, 1387, 1355, 1215, 1180, 1035, 839
NMR (DMSO-$d_6$) δ ppm: 3.80 (3H,S), 4.34 (2H,bs), 4.93 (1H,d,J=4.6), 5.51 (1H,dd, J=4.6, 8.4), 6.65 (1H,S), 7.17 (2H,bs), 7.40 (2H,d,J=7.2), 7.94 (2H,d,J=7.2), 9.46 (1H,d, J=8.4)

Example 31

Preparation of 4 Methoxybenzyl (6R,7R)-7-[(Z)-2-(2-tritylaminothiazol-4-yl)-2-ethoxyiminoacetamido]-3-(1-oxypyridin-4-yl)thiomethylceph-3-em-4-carboxylate-1-oxide.

By the same procedure as in Example 12, title compound was obtained.
$IR_\nu KBr$ max $cm^{-1}$: 2969, 1793, 1717, 1676, 1610, 1514, 1243, 1174, 1033, 826, 751, 700
NMR (CD $Cl_3$) δ ppm: 1.32 (3H,b,J=7), 3.41 (1H,d,J=18), 3.75 (3H,S), 3.92 (1H,d,J=18) 4.02 (2H,bs), 4.27 (2H,g,J=7), 4.61 (2H,d,J=4.4), 5.05 (2H,S), 5.99 (1H,dd,J=4.4,10), 6.59 (1H,S), 6.78 (2H,d,J=8.4), 7.21 (20H,m), 7.43 (1H,d,J=10) 7.80 (2H,d,J=6.8).

## Claims

1 Cephem compounds or salts thereof represented by the formula:

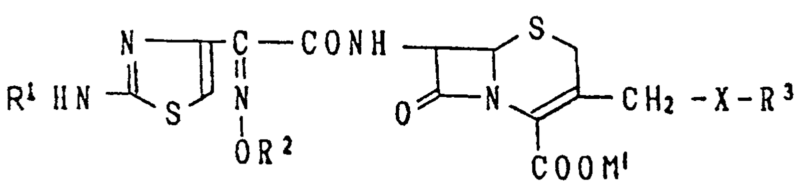

(I)

[in the formula, $R^1$ denotes hydrogen or an amino protective group, $R^2$ denotes an unsubstituted or substituted alkyl group, X denotes an O-bonded group or S-bonded group, and $M^1$ denotes hydrogen, carboxyl protective group or a cation and, as $COOM^1$, a carboxylate ion, and $R^3$ denotes a group represented by the formula:

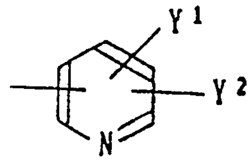

(IIa)

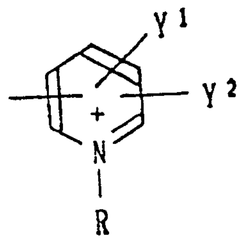

(IIb)

or

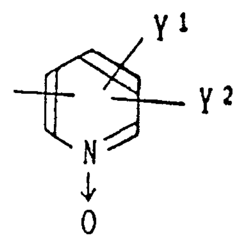

(IIc)

(in the formulae, $Y^1$ and $Y^2$ are identical or different and denote hydrogen, alkyl groups, halogen groups, hydroxyl groups or oxo groups, R denotes an alkyl group, and the bonding partner of the -X-group is positioned in the 1-position, 3-position or 4-position of the pyridine ring (but excluding the 1-position in the case of the pyridinium ring and the pyridine N-oxide ring)) (but excluding groups represented by the formula (II b) when X is an S-bonded group,].

2 Cephem compounds or salts thereof as claimed in Claim 1 in which $R^2$ is a methyl group or the 2-hydroxy-1-propyl group.

3 Cephem compounds or salts thereof as claimed in Claim 1 in which X is an O-bonded group and $R^3$ is a group in which both $Y^1$ and $Y^2$ are hydrogen or a group in which $Y^1$ is hydrogen and $Y^2$ is a 4-oxo group in formula (II a), a group in which R is an ethyl group and $Y^1$ and $Y^2$ are both hydrogen in formula (II b), or a group in which $Y^1$ and $Y^2$ are both hydrogen in formula (II c).

4 Cephem compounds or salts thereof as claimed in Claim 1 in which X is an S-bonded group, and $R^3$ is a group in which $Y^1$ and $Y^2$ are both hydrogen, or $Y^1$ is hydrogen and $Y^2$ is a fluoro group or chloro group in formula (II c).

5 A production method for cephem compounds or salts thereof as claimed in Claim 1, characterized in that cephem-1-oxide compounds represented by the formula:

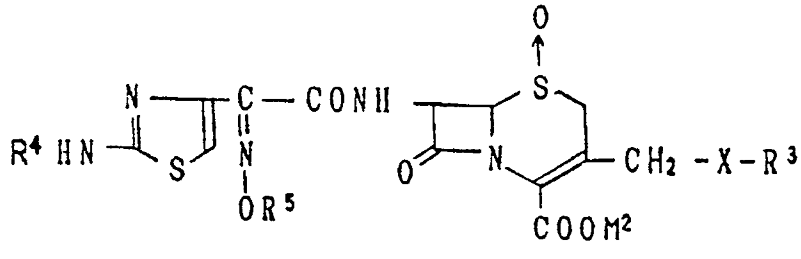

(III)

[in the formula, $R^4$ denotes an amino protective group, $R^5$ denotes an unsubstituted or substituted alkyl group (but protected by a protective group when the substituent group of the substituted alkyl group is a reactive group), $M^2$ denotes a carboxyl protective group, and X and $R^3$ respectively denote the same groups as mentioned previously] is subjected to a deoxidation reaction to obtain a cephem compound represented by the formula:

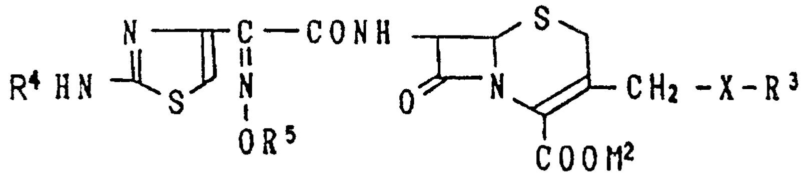
(IV)

(in the formula, $R^4$, $R^5$, $M^2$, X and $R^3$ respectively denote the same groups as mentioned previously), in other words the protective groups are eliminated.

6 A production method for cephem compounds or salts thereof as claimed in Claim 1, characterized in that 3-halogenomethylcephem compounds represented by the formula:

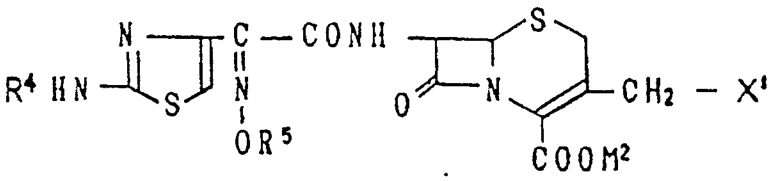
(V)

(in the formula, $X^1$ denotes a halogen group, and $R^4$, $R^5$ and $M^2$ respectively denote the same groups as mentioned previously) are reacted with hydroxy- or mercapto-pyridine derivatives represented by the formula:

$H\text{-}X\text{-}R^3$ (VI)

(in the formula, X and $R^3$ respectively denote the same groups as mentioned previously), to obtain a cephem compound represented by the formula:

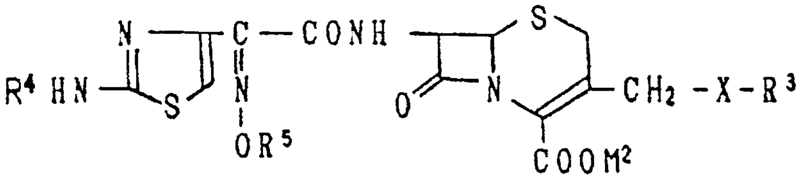
(IV)

(in the formula, $R^4$, $R^5$, $M^2$, X and $R^3$ respectively denote the same groups as mentioned previously), in other words the protective groups are eliminated.

7 A production method as claimed in Claim 6 wherein the reaction between the compound represented by formula (V) and the compound represented by the formula (VI) is carried out by means of a two-phase reaction with water and an organic solvent system using a phase transfer catalyst.

8 Cephem compounds or salts thereof represented by the formula:

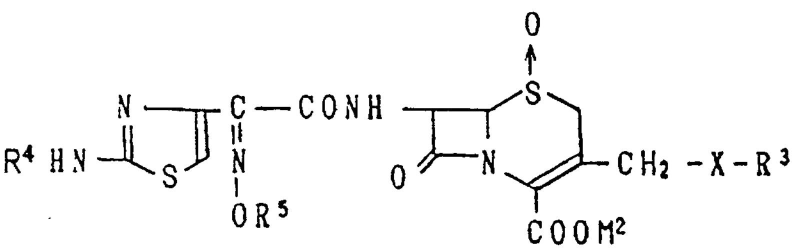
(III)

(in the formula, $R^4$, $R^5$, $M^2$, X and $R^3$ respectively denote the same groups as mentioned previously).

9 A production method for cephem compounds or salts thereof as claimed in Claim 8, characterized in that a 3-halogenomethylcephem-1-oxide compound represented by the formula:

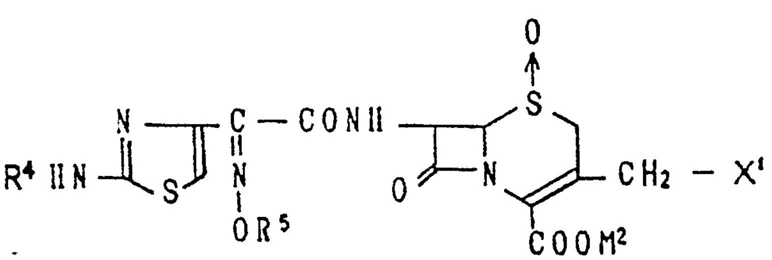
(VII)

(in the formula, $R^4$, $R^5$, $M^2$ and $X^1$ respectively denote the same groups as mentioned previously, is reacted with a hydroxy- or mercapto-pyridine derivative represented by the formula:

$H\text{-}X\text{-}R^3$ (VI)

(in the formula, X and $R^3$ respectively denote the same groups as mentioned previously).

10 A production method as claimed in Claim 9 in which the reaction between the compound represented by formula (VII) and the compound represented by the formula (VI) is carried out by means of

a two-phase reaction with water and an organic solvent system using a phase transfer catalyst.

11 Antimicrobial agents characterized in that they contain a cephem compound or salt thereof as claimed in Claim 1 as the active principle.